# EUROPÄISCHE PATENTSCHRIFT

(11) **EP 1 452 146 B1**
(45) Veröffentlichungstag und Bekanntmachung des Hinweises auf die Patenterteilung: **17.05.2006**
(21) Anmeldenummer: 04004551.0
(22) Anmeldetag: 27.02.2004
(51) Int. Cl.: A61B 17/86

(54) **Kompressionsknochenschraube**
Compression screw for osteosynthesis
Vis à compression pour l'ostéosynthèse

(30) Priorität: 27.02.2003 US 375665
(43) Veröffentlichungstag der Anmeldung: 01.09.2004
(73) Patentinhaber: Stryker Leibinger GmbH & Co. KG, 79111 Freiburg (DE)
(72) Erfinder: Rübecamp, Reinhard, 79364 Malterdingen (DE); Capanni, Felix, 79117 Freiburg (DE); Eckerle, Hans-Urs, 79117 Freiburg (DE); Forst, Peter, 79312 Emmendingen (DE)
(74) Vertreter: Röthinger, Rainer

(56) Entgegenhaltungen:
- WO-A-00/67652
- DE-A- 10 101 267
- DE-U- 20 213 235
- GB-A- 2 323 533
- US-A- 4 640 271

## Beschreibung

### GEBIET DER ERFINDUNG

Die Erfindung betrifft eine Kompressionsknochenschraube mit einem Schaft, der entlang seiner axialen Erstreckung zwei gegenüberliegende Schaftabschnitte aufweist, wobei im Bereich des ersten Schaftabschnitts ein erstes Gewinde drehfest mit dem Schaft verbunden ist und der zweite Schaftabschnitt zur drehbaren Aufnahme eines ein zweites Gewinde tragenden Bauteils ausgestaltet ist.

### HINTERGRUND DER ERFINDUNG

Kompressionsknochenschrauben werden bei Operationen eingesetzt, um zwei Bruchstücke eines gebrochenen Knochens unter Kompression zusammenzufügen. Die Kompression unterstützt das Zusammenwachsen der beiden Bruchstücke.

Eine derartige Kompressionsknochenschraube ist aus der US 4,858,601 bekannt. Es handelt sich um eine doppelgewindige Schraube, welche aus drei separaten Bauteilen besteht. Zwei dieser Bauteile tragen jeweils ein Gewinde mit gleicher Gewindesteigung und gleichem Gewindedurchmesser. Die beiden mit einem Gewinde versehenen Gewindebauteile sind jeweils als hohlzylindrische Hülse ausgestaltet.

Das dritte Bauteil der Kompressionsknochenschraube ist ein stiftförmiger Schaft, der entlang seine axialen Erstreckung in zwei gegenüberliegende Schafthälften untergliedert ist und an einem seiner beiden Enden mit einer Durchmessererweiterung versehen ist. Auf den Schaft werden nacheinander die beiden hülsenförmigen Gewindebauteile derart aufgesteckt, dass die Durchmessererweiterung des Schafts als Anschlag für die beiden Gewindebauteile fungiert. Anschließend wird das zuletzt auf den Schaft aufgesteckte Gewindebauteil durch Löten drehfest mit dem Schaft verbunden. Das andere der beiden Gewindebauteile ist hingegen frei um den Schaft als Drehachse drehbar.

Sowohl die Durchmessererweiterung des Schafts als auch das bezüglich des Schafts drehbare Gewindebauteil sind jeweils mit einer Kraftaufnahmestruktur in Gestalt eines Schlitzes versehen. Die Schlitze ermöglichen das getrennte Einleiten eines Drehmoments in das drehbare Gewindebauteil sowie in das drehfest mit dem Schaft verbundene Gewindebauteil.

Zum Befestigen der Schraube werden zwei Schraubendreher mit jeweils unterschiedlicher Klinge benutzt. Die Klinge des ersten der beiden Schraubendreher ist zum Zusammenwirken mit dem Schlitz in der Durchmessererweiterung des Schafts ausgebildet und die Klinge des zweiten der beiden Schraubendreher zum gleichzeitigen Zusammenwirken mit dem Schlitz in dem drehbaren Gewindebauteil sowie mit dem Schlitz in der Durchmessererweiterung des Schafts. Während der zweite Schraubendreher folglich das Eindrehen der Knochenschraube als Ganzes gestattet, ermöglicht der erste Schraubendreher eine Relativdrehung zwischen den beiden Gewindebauteilen.

Aus der US 4,640,271 ist eine zweiteilige Kompressionsknochenschraube bekannt. Die Knochenschraube umfasst einen Schaft, der im Bereich eines ersten Schaftabschnittes mit einem Gewinde versehen ist und an einem dem Gewinde gegenüberliegenden zweiten Schaftabschnitt zwei bewegliche Rastzungen aufweist. Die Rastzungen ermöglichen es, ein separates Gewindebauteil mit dem Schaft zu koppeln. In der Praxis hat sich herausgestellt, dass es bei hohen Kompressionskräften zu einem ungewollten Lösen des Gewindebauteils von den Rastzungen und damit vom Schraubenschaft kommen kann.

Aus der DE 101 01 267 A1 (Basis für den Oberbegriff des Anspruchs 1) ist eine weitere zweiteilige Kompressionsknochenschraube mit Rastzungen bekannt.

Die DE 202 13 235 U1 betrifft einen Ostheosynthese-Nagel mit einem Schaft und einem gewindetragenden drehbaren Kopfteil. Zur Kopplung zwischen Kopfteil und Nagelschaft ist ein Sprengring vorgesehen, der in gegenüberliegenden halbkreisförmigen Nuten auf der Innenseite des Kopfteils und auf der Außenseite des Nagelschafts aufgenommen ist.

Der Erfindung liegt die Aufgabe zugrunde, eine Kompressionsknochenschraube anzugeben, bei der sichergestellt ist, dass auch bei hohen Kompressionskräften kein Lösen des Gewindebauteils vom Schraubenschaft auftreten kann.

### ZUSAMMENFASSUNG DER ERFINDUNG

Diese Aufgabe wird gelöst durch eine Kompressionsknochenschraube mit den Merkmalen des Anspruchs 1.

Es wird eine Kompressionsknochenschraube vorgeschlagen mit einem Schaft, der entlang seiner axialen Erstreckung zwei gegenüberliegende Schaftabschnitte aufweist, wobei im Bereich des ersten Schaftabschnittes ein erstes Gewinde drehfest mit dem Schaft verbunden ist und der zweite Schaftabschnitt zur drehbaren Aufnahme eines Gewindebauteils, welches ein zweites Gewinde trägt, ausgebildet ist, und wobei zusätzlich ein bezüglich der axialen Erstreckung des Schraubenschafts in radialer Richtung verformbares Koppelelement in Form eines losen Sicherungsrings mit sich in radialer Richtung erstreckenden Stirnflächenabschnitten zum Koppeln des Gewindebauteils mit dem Schaft vorhanden ist.

Die erfindungsgemäße Kompressionsknochenschraube ist mehrteilig, d.h. sie umfasst neben dem Schaft und dem Gewindebauteil ein vorteilhafterweise loses Koppelelement. Das Koppelelement ist in radialer Richtung verformbar, beispielsweise aufweitbar und/oder komprimierbar. Das Koppelelement ist vorzugsweise derart ausgelegt, dass es bezüglich einer Umfangsrichtung der Knochenschraube Kompressionskräfte zwischen dem Schaft und dem Gewindebauteil zumindest annähernd gleichmäßig überträgt. Das Koppelelement kann daher bezüglich einer Schraubenlängsachse rotationssymetrisch bzw. annähernd rotationssymetrisch ausgebildet sein. Es wäre auch denkbar, dass das Doppelelement aus einer Mehrzahl einzelner Elemente besteht, die in Umfangsrichtung der Knochenschraube derart verteilt sind, dass es zu einer im wesentlichen rotationssymetrischen Übertragung von Kompressionskräften zwischen Schaft und Gewindebauteil kommt.

Das Koppelelement kann viele verschiedene Formen besitzen. So kann das Koppelelement beispielsweise ringförmig, ringsegmentförmig, usw. ausgebildet sein. Ein Sprengring kann als ringsegmentförmiges Koppelelement dienen.

Das Koppelelement kann ein rastendes Koppeln des Gewindebauteils mit dem Schaft ermöglichen. Zu diesem Zweck ist es denkbar, dass der Schaft radial außen mit wenigstens einer Vertiefung zur rastenden Aufnahme des Koppelelements versehen ist. Zusätzlich oder alternativ hierzu ist es möglich, das Gewindebauteil radial innen mit wenigstens einer Vertiefung zur rastenden Aufnahme des Koppelelements zu versehen.

Der Schaft, das Gewindebauteil oder beide Komponenten können Mittel zum Verformen des Koppelelements aufweisen. Derartige Mittel sind beim Gewindebauteil zweckmäßigerweise radial innen und beim Schaft zweckmäßigerweise radial außen angeordnet. Die Mittel zum Verformen des Koppelelements können beispielsweise als Flanken, Schrägen, konische Abschnitte usw. ausgebildet sein. Die Mittel zum Verformen des Koppelelements können eine Bewegung des Koppelelements radial nach außen und/oder radial nach innen bewirken.

Wenigstens eines der beiden Gewinde kann einen sich entlang der axialen Erstreckung der Knochenschraube veränderlichen Gewindedurchmesser besitzen. So kann das erste Gewinde einen in Richtung auf das Gewindebauteil zunehmenden Gewindedurchmesser aufweisen.

Das am Schaft ausgebildete erste Gewinde kann einen geringeren Durchmesser als das zweite Gewinde des Gewindebauteils aufweisen. Zusätzlich kann der Schaft zwischen dem ersten und dem zweiten Schaftabschnitt Schneidkanten besitzen, welche eine Vorbohrfunktion bezüglich des zweiten Gewindes mit dem größeren Durchmesser erfüllen.

Obwohl der Schraubengrundkörper grundsätzlich einen zweiteiligen Aufbau aus dem Schaft und einem das erste Gewinde tragenden, separaten Gewindebauteil aufweisen kann, ist gemäß einer bevorzugten Ausführungsform der Erfindung das erste Gewinde einstückig mit dem Schaft ausgebildet.

Das Gewindebauteil ist vorzugsweise als Hülse ausgestaltet, welche entweder eine durchgehende Öffnung aufweist oder an einem der beiden Hülsenenden verschlossen ist. Für den Fall, dass beide Hülsenenden offen sind und das Gewindebauteil somit eine durchgehende Öffnung aufweist, kann auch der Schaft mit einer durchgehenden, sich in axialer Richtung erstreckenden Öffnung versehen sein. Somit wird ermöglicht, einen Zieldraht durch die Kompressionsknochenschraube hindurch zu führen. Derartige Zieldrähte werden mittels einer Bohrmaschine direkt in beide zu verbindende Knochenfragmente gebohrt und gestatten eine definierte Platzierung der Kompressionsknochenschraube. Als Zieldrähte werden üblicherweise sog. Kirschnerdrähte mit einem Durchmesser von ungefähr einem Millimeter verwendet.

Gemäß einer bevorzugten Ausführungsform besitzt das erste Gewinde einen kleineren Durchmesser als das zweite Gewinde. Weiterhin kann die erfindungsgemäße Kompressionsknochenschraube mittels eines entsprechenden Anschliffs der Schraubenspitze selbstbohrend und können die beiden Gewinde mittels einer entsprechend spitz gestalteten Geometrie der Gewindeflanken selbstschneidend ausgebildet sein. Wird eine derart ausgestaltete Kompressionsknochenschraube im Knochen versenkt, so schneidet sich zuerst das erste Gewinde mit dem geringeren Durchmesser in den Knochen. Ist die Kompressionsknochenschraube soweit eingedreht, dass bereits das zweite Gewinde greift, so wird die von dem ersten Gewinde erzeugte Gewindebohrung im Knochen durch Verdrängung aufgeweitet und vom zweiten Gewinde neu geschnitten.

Der Schaft kann zwischen dem ersten und dem zweiten Schaftabschnitt mit einer Durchmessererweiterung versehen sein, welche in Richtung auf das erste Gewinde als Anschlag für das Gewindebauteil fungiert. Für den Fall, dass das erste Gewinde einen kleineren Durchmesser als das zweite Gewinde aufweist, kann die Durchmessererweiterung eine in Richtung auf das erste Gewinde kontinuierlich abnehmenden Durchmesser, d.h. eine konische Bauform aufweisen. Vorzugsweise ist die Durchmessererweiterung radial außen mit den Schneidkanten versehen, welche die Vorbohrfunktion bezüglich des zweiten Gewindes mit dem größeren Durchmesser besitzen. So können beispielsweise zwei bzw. drei Schneidkanten vorgesehen werden, welche sich jeweils über 180° bzw. 120° des Außenumfangs der Durchmessererweiterung erstrecken. Vorzugsweise werden fünf oder mehr Schneidkanten verwendet.

Der Schaft oder das erste Gewinde kann mit einer ersten Kraftaufnahmestruktur versehen sein, welche das Einleiten einer Kraft und insbesondere eines Drehmoments in den Schaft oder das erste Gewinde gestattet. Zum gleichen Zweck kann das Gewindebauteil eine zweite Kraftaufnahmestruktur besitzen. Die erste bzw. zweite Kraftaufnahmestruktur kann beispielsweise schlitzartig, als Rippe, als Innenmehrkant, als Außenmehrkant usw. ausgestaltet sein. Besonders vorteilhaft ist eine Kraftaufnahmestruktur in Gestalt eines Kreuzschlitzes oder in Gestalt eines Innenmehrkants. Das Vorsehen getrennter Kraftaufnahmestrukturen für das Gewindebauteil sowie den Schaft oder das erste Gewinde gestattet das getrennte Einleiten eines Drehmoments.

Zum Einleiten einer Kraft in die Kraftaufnahmestrukturen der Kompressionsknochenschraube ist vorzugsweise ein Schraubendreher mit einer Schraubendreherklinge vorgesehen, welche eine erste Krafteinleitungsstruktur, eine sich durch die erste Krafteinleitungsstruktur erstreckende zweite Krafteinleitungsstruktur sowie einen Koppelmechanismus, welcher in einer ersten Stellung die erste Krafteinleitungsstruktur drehfest mit der zweiten Krafteinleitungsstruktur verbindet und einer zweiten Stellung eine Relativdrehung zwischen der ersten Krafteinleitungsstruktur und der zweiten Krafteinleitungsstruktur gestattet, umfasst. Sowohl die erste Krafteinleitungsstruktur als auch die zweite Krafteinleitungsstruktur sind vorteilhafterweise jeweils derart ausgestaltet, dass eine Krafteinleitung in jeweils eine der Kraftaufnahmestrukturen der Kompressionsknochenschraube möglich ist. Die Schraubendreherklinge kann zwei Krafteinleitungsstrukturen in Gestalt z. B. jeweils einer Schneide, beispielsweise einer Kreuzschneide, oder eines Außenmehrkants aufweisen, welche jeweils mit einer komplementären Kraftaufnahmestruktur der Kompressionsknochenschraube derart zusammenwirken, dass ein Drehmoment in die Knochenschraube eingeleitet werden kann.

Im Gegensatz zum Stand der Technik muss zum Versenken der erfindungsgemäßen Kompressionsknochenschraube somit nicht mehr mit zwei separaten Schraubendrehern gearbeitet werden. Vielmehr ermöglicht die erfindungsgemäße Schraubendreherklinge sowohl das Versenken der Kompressionsknochenschraube als Ganzes als auch ein getrenntes Drehen von Schaft und Gewindebauteil, ohne dass ein Schraubendreherwechsel von Nöten wäre.

Erfindungsgemäß wird in der ersten Stellung des Koppelmechanismus die Kompressionsknochenschraube zunächst als Ganzes mittels des Schraubendrehers im Knochen versenkt. Anschließend findet in der zweiten Stellung des Koppelmechanismus ein weiteres Eindrehen des ersten Gewindes oder ein Zurückdrehen des Gewindebauteils statt, um eine Kompression zu erzielen.

Es ist zweckmäßig, einen Selbsthaltemechanismus vorzusehen, welcher gewährleistet, dass die Kompressionsknochenschraube nicht ungewollt von der Schraubendreherklinge abfällt. So ist es denkbar, die sog. Friction Fit-Technik zu verwenden, welche auf einem Reibschluss zwischen Schraubendreherklinge und Kompressionsknochenschraube beruht. Anstelle eines Reibschlusses kann die Schraubendreherklinge auch mittels einer Rastverbindung mit der Kompressionsknochenschraube verbunden werden. Die Rastverbindung kann beispielsweise derart ausgestaltet sein, dass die Schraubendreherklinge an ihrem der Kompressionsknochenschraube zugewandten Ende eine ringförmige Wulst aufweist, welche mit dem mindestens einen Rastelement des Schafts der Kompressionsknochenschraube zusammenwirkt. Beim Aufsetzen der Schraubendreherklinge auf die Kompressionsknochenschraube kann das mindestens eine Rastelement des Schafts von der Wulst radial nach außen gedrängt werden, bis die ringförmige Wulst der Schraubendreherklinge im Bereich eines Hinterschnitts des Rastelements angeordnet ist. Das Rastelement kann sich daraufhin in seine Ausgangsposition zurückbewegen.

Wie die Kompressionsknochenschraube kann auch der Schaft des Schraubendrehers entlang seiner axialen Erstreckung und somit auch die Schraubendreherklinge eine durchgehende Öffnung aufweisen, um die Verwendung des oben beschriebenen Zieldrahtes beim Versenken der Kompressionsknochenschraube zu ermöglichen.

### KURZE BESCHREIBUNG DER FIGUREN

Vorteilhafte Weiterbildungen und Ausgestaltungen der Erfindung ergeben sich aus den Figuren und dem Ausführungsbeispiel. Es zeigen:
- Fig. 1A: eine Schnittansicht eines Ausführungsbeispiels einer erfindungsgemäßen Kompressionsknochenschraube;
- Fig. 1B: das Gewindebauteil der erfindungsgemäßen Kompressionsknochenschraube;
- Fig. 1C: den Schaft der erfindungsgemäßen Kompressionsknochenschraube; und
- Fig. 2A-2B: den Vorgang des Zusammenbauens der erfindungsgemäßen Kompressionsknochenschraube.

### BESCHREIBUNG EINES BEVORZUGTEN AUSFÜHRUNGSBEISPIELS

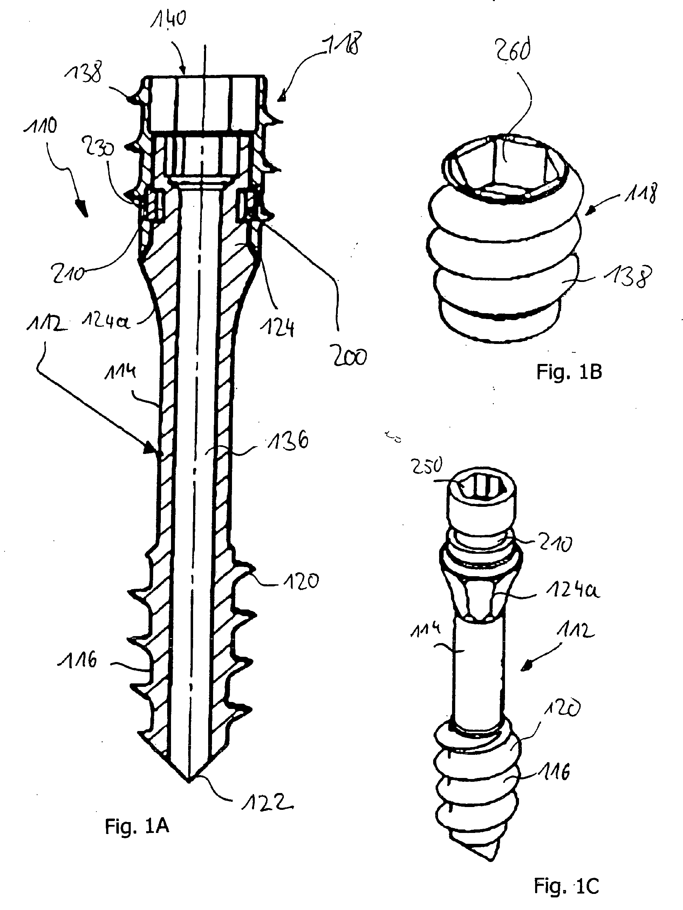

In den Figuren 1A bis 1C sind ein Ausführungsbeispiel einer erfindungsgemäßen kanülierten Kompressionsknochenschraube 110 in einer Schnittansicht sowie Ansichten zweier Einzelteile der Kompressionsknochenschraube 110 dargestellt.

Die dreiteilige Kompressionsknochenschraube 110 umfasst einen Schaft 112 mit zwei gegenüberliegenden Schaftabschnitten 114, 116, ein separates Gewindebauteil 118 sowie ein loses Koppelelement 200.

Der Schaft 112 weist im Bereich des ersten Schaftabschnitts 116 ein Außengewinde 120 auf, welches einstückig mit dem Schaft 112 ausgebildet ist. Das Gewinde 120 ist selbstschneidend ausgeführt und mündet in eine Schraubenspitze 122, welche selbstbohrend angeschliffen ist. Der dem ersten Schaftabschnitt 116 gegenüberliegende zweite Schaftabschnitt 114 ist mit einer konischen Durchmessererweiterung 124 versehen. Radial außen ist die konische Durchmessererweiterung 124 mit mehreren Schneidkanten 124a versehen, welche eine Vorbohrfunktion bezüglich eines Gewindes 138 des Gewindebauteils 118 aufweisen.

Wie sich aus den Fign. 1B und 1C ergibt, sind der Schaft 112 und das Gewindebauteil 118 mit jeweils einer Krafteinleitungsstruktur in Gestalt eines Innensechskants 250, 260 versehen. Der Innensechskant 260 des Gewindebauteils 118 besitzt einen größeren Durchmesser als der Innensechskant 250 des Schafts 112. Wie aus Fig. 1A ersichtlich, sind die beiden Innensechskante 250, 260 bei fertig montierter Kompressionsknochenschraube 110 konzentrisch zueinander, aber in axialer Richtung beabstandet voneinander derart angeordnet, dass sich eine mit dem Innensechskant 250 des Schafts 112 zusammenwirkende Schraubendreherklinge durch das Gewindebauteil 118 und dessen Innensechskant 260 hindurch erstreckt. Diese Anordnung ermöglicht es einem Chirurgen, mittels geeigneter Schraubendreherklingen ein Drehmoment wahlweise in den Schraubenschaft 112 oder in das Gewindebauteil 118 einzuleiten. Auf eine genauere Schilderung der chirurgischen Vorhergehensweise wird hier verzichtet, da sie in vielen Aspekten mit der Vorhergehensweise bei bekannten Kompressionsknochenschrauben übereinstimmt.

Wie Fig. 1A entnommen werden kann, weist der Schaft 112 eine entlang der Längsachse des Schafts 112 verlaufende, durchgehende Öffnung 136 zum Zweck der Aufnahme eines Zieldrahts auf. Der Schaft 112 ist also kanüliert.

Das Gewindebauteil 118 der in den Figuren 1A und 1B dargestellten Kompressionsknochenschraube 110 ist mit einem selbstschneidenden Außengewinde 138 versehen. Das Gewinde 138 des Gewindebauteils 118 ist zylindrisch geformt, d. h. der Gewindedurchmesser ist über die Länge des Gewindes 138 konstant. Der Durchmesser des Gewindes 138 des Gewindebauteils 118 ist größer als der Durchmesser des Gewindes 116 des Schafts 112.

Wie der Schaft 112 besitzt auch das Gewindebauteil 118 eine durchgehende Öffnung 140. Das Gewindebauteil 118 ist demnach hülsenförmig ausgestaltet und gestattet das Durchführen eines Zieldrahts durch das Gewindebauteil 118 hindurch.

Eine wesentliche Neuerung bei der Knochenschraube 110 liegt im Verbindungsmechanismus des Schafts 112 mit dem separaten Gewindebauteil 118. Während bei herkömmlichen Knochenschrauben die Verbindung von Schaft und Gewindebauteil mittels Schnapphaken, Rastzungen oder ähnlichen Mitteln bewerkstelligt wurde, kommt jetzt als zusätzliches Bauteil ein bezüglich der axialen Erstreckung der Schraube 110 in radialer Richtung verformbares, loses Koppeldement in Gestalt z. B. eines Sicherungsringes 200 zum Einsatz. Es hat sich gezeigt, dass damit wesentlich höhere Kompressionskräfte übertragen werden können, ohne dass es zu einem unerwünschten Lösen des Gewindebauteils 118 vom Schaft 112 kommt.

Eine weitere Eigenart der Knochenschraube 110 betrifft das Schraubenfußgewinde 120. Es hat sich gezeigt, dass bei einer Doppelgewindeschraube, bei der das Gewinde des Gewindebauteils einen größeren Durchmesser als das Gewinde des Schafts besitzt, beim Herausdrehen der Schraube in gewissen Situationen Probleme auftreten. Daher ist bei der Kompressionsknochenschraube 110 der Durchmesser der oberen Gewindegänge des Gewindes 120 des Schafts 112 vergrößert (in den Figuren nicht erkennbar). Diese Durchmesservergrößerung erhöht das Eindrehmoment nur sehr gering, erleichtert andererseits aber das Entfernen der Knochenschraube 110 aus dem Knochen ganz wesentlich.

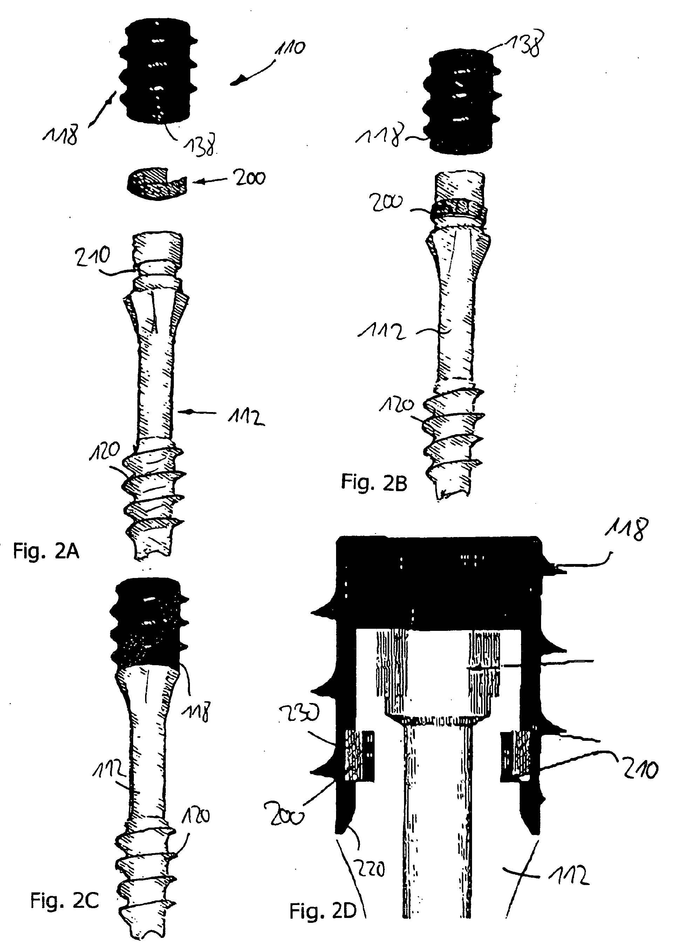

Nachfolgend wird unter Bezugnahme auf die Figuren 1B, 1C und 2A bis 2D der Zusammenbau einer Kompressionsknochenschraube 110 erläutert.

Wie sich aus Fig. 2A ergibt, besteht die kanülierte Kompressionsknochenschraube 110 aus drei Bauteilen, nämlich einem Schraubenfuß in Gestalt des Schafts 112 mit dem Gewinde 120, einem Schraubenkopf in Gestalt eines separaten Gewindebauteils 118 mit einem Gewinde 138 sowie einem Koppelelement in Gestalt des aufweitbaren (und im Ausführungsbeispiel nicht geschlossenen) Sicherungsringes 200. Beide Gewinde 120, 138 besitzen die gleiche Steigung sowie die gleiche Gewinderichtung.

In einem ersten Schritt (Fig. 2B) wird der nach Art eines Sprengrings radial aufweitbare Sicherungsring 200 in eine hierfür vorgesehene, am Schaft 112 ausgebildete und in Umfangsrichtung des Schafts 112 verlaufende Nut 210 eingebracht. Hierfür ist zunächst eine geringfügige, elastische Aufbreitung des Sicherungsrings 200 notwendig, wenn dieser auf den Schaft 112 aufgeschoben wird. Sobald sich der Sicherungsring 200 in axialer Richtung im Bereich der Nut 210 befindet, bewirken die elastischen Eigenschaften des Sicherungsrings 200, dass sich dieser wieder zusammenzieht und dabei in die Nut 210 einrastet und unverlierbar in der Nut 210 aufgenommen ist.

Wie Fig. 2B entnommen werden kann, ist der Innendurchmesser des Sicherungsrings 200 geringfügig größer als der Außendurchmesser des Grunds der Nut 210. Der Sicherungsring 200 ist daher in Richtung auf den Grund der Nut 210 verformbar. Weiterhin ist der Außendurchmesser des Sicherungsrings 200 etwas größer als der Außendurchmesser desjenigen Bereichs des Schafts 112, der an die Nut 210 in axialer Richtung angrenzt. Mit anderen Worten, das Koppelelement in Gestalt des Ringes 200 steht in radialer Richtung etwas über den Schaft 112 hervor und kann außerdem in radialer Richtung nach innen, d.h. in Richtung auf den Grund der Nut 210 elastisch verformt werden.

Das Gewindebauteil 118 ist auf den Schaft 112 aufsteckbar. Beim Aufstecken des Gewindebauteils 118 wirken konische Flanken 220 am Innendurchmesser des Gewindebauteils 118 derart mit dem Sicherungsring 200 zusammen, dass dieser radial nach innen verformt wird und dabei einen kleineren Durchmesser annimmt. Das Gewindebauteil 118 besitzt radial innen eine in Umfangrichtung des Gewindebauteils 118 verlaufende Nut 230, in welche der Sicherungsring 200 rastend expandieren kann, sobald beim Aufstecken des Gewindebauteils 118 die axiale Position des Sicherungsrings 200 mit der axialen Position der Nut 230 des Gewindebauteils 118 übereinstimmt.

Nach der radialen Expansion des Sicherungsrings 200 ist das Gewindebauteil 118 mittels des gleichzeitig in die Nut 210 und die Nut 230 eingreifenden Sicherungsringes 200 unverlierbar mit dem Schaft 112 gekoppelt. Etwaige Kompressionskräfte zwischen dem Gewindebauteil 118 und dem Schaft 112 werden mittels des Sicherungsrings 200 zuverlässig übertragen. Insbesondere ringförmige oder annähernd ringförmige Koppelelemente wie der als Springring ausgebildete und nur teilweise geschlossene Sicherungsring 200 ermöglichen eine in Umfangsrichtung der Knochenschraube gleichmäßige Übertragung von Kompressionskräften zwischen dem Schaft 112 und dem Gewindebauteil 118.

Bei der erfindungsgemäßen Kompressionsknochenschraube 110 kommt es auch bei hohen Kompressionskräften zu keinem Lösen des Gewindebauteils 118 vom Schaft 112. Ursache hierfür ist das unter Bezugnahme auf das Ausführungsbeispiel beispielhaft beschriebene Koppelungskonzept unter Verwendung eines in radialer Richtung verformbaren Koppelelements. Dieses Koppelungskonzept ist herkömmlichen Koppelungskonzepten, wie der aus der US 4,640,271 bekannten Verwendung einzelner Rastzungen, an Stabilität deutlich überlegen.

## Patentansprüche

1. Kompressionsknochenschraube (110) mit einem Schaft (112), der entlang seiner axialen Erstreckung zwei gegenüberliegende Schaftabschnitte (114, 116) aufweist, wobei im Bereich eines ersten Schaftabschnittes (114) ein erstes Gewinde (120) drehfest mit dem Schaft (112) verbunden ist und ein zweiter Schaftabschnitt (116) zur drehbaren Aufnahme eines Gewindebauteils (118), welches ein zweites Gewinde (138) trägt, ausgebildet ist,
**dadurch gekennzeichnet, dass** ein in radialer Richtung verformbares Koppelelement in Form eines losen Sicherungsrings (200) zum Koppeln des Gewindebauteils (118) mit dem Schaft (112) vorgesehen ist, wobei der Sicherungsring (200) sich in radialer Richtung erstreckende Stirnflächenabschnitte besitzt.

2. Kompressionsknochenschraube nach Anspruch 1,
**dadurch gekennzeichnet, dass** der Sicherungsring (200) einen rechteckigen Querschnitt besitzt.

3. Kompressionsknochenschraube nach Anspruch 1 oder 2, **dadurch gekennzeichnet, dass** der Sicherungsring ein geschlitzter Springring (200) ist.

4. Kompressionsknochenschraube nach einem der Ansprüche 1 bis 3,
**dadurch gekennzeichnet, dass** der Schaft (112) radial außen mit wenigstens einer Vertiefung (210) zur rastenden Aufnahme des Sicherungsrings (200) versehen ist.

5. Kompressionsknochenschraube nach einem der Ansprüche 1 bis 4,
**dadurch gekennzeichnet, dass** das Gewindebauteil (118) radial innen mit wenigstens einer Vertiefung (230) zur rastenden Aufnahme des Sicherungsrings (200) versehen ist.

6. Kompressionsknochenschraube nach Anspruch 4 oder 5,
**dadurch gekennzeichnet**, das die Vertiefung (210) des Schafts (112) und/oder die Vertiefung (230) des Gewindebauteils (118) durch eine sich in radialer Richtung erstreckende Wand begrenzt ist/sind, die mit jeweils einem Stirnflächenabschnitt des Sicherungsrings zusammen wirkt/wirken.

7. Kompressionsknochenschraube nach einem der Ansprüche 1 bis 6,
**dadurch gekennzeichnet, dass** der Schaft (112) radial außen Mittel zum Verformen des Sicherungsrings (200) aufweist.

8. Kompressionsknochenschraube nach einem der Ansprüche 1 bis 7,
**dadurch gekennzeichnet, dass** das Gewindebauteil (118) radial innen Mittel (220) zum Verformen des Sicherungsrings (200) aufweist.

9. Kompressionsknochenschraube nach einem der Ansprüche 1 bis 8,
**dadurch gekennzeichnet, dass** das erste Gewinde (120) einen in Richtung auf das Gewindebauteil (118) zunehmenden Gewindedurchmesser aufweist.

10. Kompressionsknochenschraube nach einem der Ansprüche 1 bis 9,
**dadurch gekennzeichnet, dass** das erste Gewinde (120) einen geringeren Durchmesser als das zweite Gewinde (138) aufweist und der Schaft (112) zwischen dem ersten und dem zweiten Schaftabschnitt (114, 116) Schneidkanten (124a) besitzt, die eine Vorbohrfunktion bezüglich des zweiten Gewindes (138) mit dem größeren Durchmesser besitzen.

## Claims

1. A compression bone screw (110) with a shank (112) having two opposite shank sections (114, 116) along its axial extent, a first thread (120) being connected in a rotationally fixed manner to the shank (112) in the region of the first shank section (114), and a second shank section (116) being designed for rotatable reception of a threaded component (118) bearing a second thread (138),
**characterized in that** a coupling element embodied by a loose securing ring (200) deformable in radial direction is provided for coupling the threaded component (118) to the shank (112), the securing ring (200) having face sections that extend in radial direction.

2. Compression bone screw according to Claim 1, **characterized in that** the securing ring (200) has a rectangular cross-section.

3. Compression bone screw according to Claim 1 or 2, **characterized in that** the securing ring is a slotted spring ring (200).

4. Compression bone screw according to one of claims 1 to 3, **characterized in that** the shank (112) is provided radially on the outside with at least one indentation (210) for the latching reception of the securing ring (200).

5. Compression bone screw according to one of claims 1 to 4, **characterized in that** the threaded component (118) is provided radially at the inside with at least one indentation (230) for the latching reception of the securing ring (200).

6. Compression bone screw according to Claim 4 or 5, **characterized in that** the indentation (210) of the shank (112) and/or the indentation (230) of the threaded component (118) is/are defined by a wall extending in radial direction and cooperating with one face section of the securing ring each.

7. Compression bone screw according to one of Claims 1 to 6, **characterized in that** the shank (112) radially at the outside is provided with means for deforming the securing ring (200).

8. Compression bone screw according to one of Claims 1 to 7, **characterized in that** the threaded component (118) radially at the inside is provided with means (220) for deforming the securing ring (200).

9. Compression bone screw according to one of Claims 1 to 8, **characterized in that** the first thread (120) has a thread diameter increasing in the direction of the threaded component (118).

10. Compression bone screw according to one of claims 1 to 9, **characterized in that** the first thread (120) has a smaller diameter than the second thread (138) and the shank (112) has, between the first and the second shank section (114, 116), cutting edges (124a) which have a predrilling function with respect to the second thread (138) with the greater diameter.

## Revendications

1. Vis à compression pour l'ostéosynthèse (110) qui présente une tige (112), laquelle possède le long de son axe d'extension deux segments de tige (114, 116) opposés, un premier filetage (120) étant relié fixe en rotation avec ladite tige (112) dans la zone d'un premier segment de tige (114) et un second segment de tige (116) étant formé pour l'accouplement en rotation d'un élément fileté (118) portant un second filetage (138), **caractérisée en ce qu'**un élément d'accouplement déformable en direction radiale et se présentant sous la forme d'un circlip (200) est prévu pour l'accouplement de l'élément fileté (118) avec la tige (112), le circlip (200) possédant des segments de surface qui s'étendent en direction radiale.

2. Vis à compression pour l'ostéosynthèse selon la revendication 1,
**caractérisée en ce que** le circlip (200) possède une section rectangulaire.

3. Vis à compression pour l'ostéosynthèse selon la revendication 1 ou 2,
**caractérisée en ce que** le circlip est une bague élastique ouverte (200).

4. Vis à compression pour l'ostéosynthèse selon l'une des revendications 1 à 3,
**caractérisée en ce que** la tige (112) est munie sur sa circonférence extérieure d'au moins un évidement (210) ménagé en direction radiale et destiné au logement par emboîtement du circlip (200).

5. Vis à compression pour l'ostéosynthèse selon l'une des revendications 1 à 4,
**caractérisée en ce que** l'élément fileté (118) est muni sur sa circonférence intérieure d'au moins un évidement (230) ménagé en direction radiale et destiné au logement par emboîtement du circlip (200).

6. Vis à compression pour l'ostéosynthèse selon la revendication 4 ou 5,
**caractérisée en ce que** l'évidement (210) ménagé dans la tige (112) et/ou l'évidement (230) ménagé dans l'élément fileté (118) est/sont limité(s) par une paroi s'étendant en direction radiale, laquelle coopère respectivement avec un segment de surface du circlip.

7. Vis à compression pour l'ostéosynthèse selon l'une des revendications 1 à 6,
**caractérisée en ce que** la tige (112) présente sur sa circonférence extérieure, en direction radiale, des moyens pour la déformation du circlip (200).

8. Vis à compression pour l'ostéosynthèse selon l'une des revendications 1 à 7,
**caractérisée en ce que** l'élément fileté (118) présente sur sa circonférence intérieure, en direction radiale, des moyens (220) pour la déformation du circlip (200).

9. Vis à compression pour l'ostéosynthèse selon l'une des revendications 1 à 8,
**caractérisée en ce que** le premier filetage (120) présente un diamètre de filetage allant croissant en direction de l'élément fileté (118).

10. Vis à compression pour l'ostéosynthèse selon l'une des revendications 1 à 9,
**caractérisée en ce que** le premier filetage (120) présente un diamètre plus faible que le second filetage (138) et la tige (112) entre le premier et le second segment de tige (114, 116) possède des arêtes coupantes (124a) dotées d'une fonction de préforage en vue du second filetage (138) de diamètre supérieur.
